# EUROPÄISCHE PATENTSCHRIFT

(11) **EP 3 304 614 B1**
(45) Veröffentlichungstag und Bekanntmachung des Hinweises auf die Patenterteilung: **05.02.2020**
(21) Anmeldenummer: 15723941.9
(22) Anmeldetag: 20.05.2015
(51) Int. Cl.: H01M 2/10, A61B 17/88, B25F 5/00, H01M 2/30, H01M 2/34, H01M 2/02

(54) **ELEKTRISCHE ENERGIEQUELLE, WERKZEUGSET UND VERFAHREN ZUM EINSETZEN EINER ENERGIEQUELLE IN EIN WERKZEUG**
ELECTRICAL ENERGY SOURCE, TOOL KIT, AND METHOD FOR INSERTING AN ENERGY SOURCE INTO A TOOL
SOURCE D'ÉNERGIE ÉLECTRIQUE, ENSEMBLE D'OUTILS ET PROCÉDÉ DE MISE EN OEUVRE D'UNE SOURCE D'ÉNERGIE DANS UN OUTIL

(43) Veröffentlichungstag der Anmeldung: 11.04.2018
(73) Patentinhaber: Medartis Holding AG, 4057 Basel (CH)
(72) Erfinder: MULLIS, Andreas, 4465 Hemmiken (CH); ZEUNER, Hermann, 79111 Freiburg (DE); SCHONHARDT, Jürgen, 79618 Rheinfelden (DE); HUBER, Micha, CH-8409 Winterthur (CH)
(74) Vertreter: Hepp Wenger Ryffel AG
(86) Internationale Anmeldenummer: PCT/EP2015/061053
(87) Internationale Veröffentlichungsnummer: WO 2016/184509

(56) Entgegenhaltungen:
- EP-A1- 2 169 750
- DE-U1-202008 001 713
- US-A1- 2012 071 711

## Beschreibung

Die vorliegende Erfindung befasst sich mit elektrischen Energiequellen für elektrisch antreibbare Werkzeuge, Werkzeugsets mit mindestens einem elektrisch antreibbaren Werkzeug und mindestens einer elektrischen Energiequelle sowie Verfahren zum Einsetzen einer Energiequelle in ein elektrisch antreibbares Werkzeug. Elektrisch antreibbare Werkzeuge benötigen zum Antrieb eine elektrische Energiequelle, die beispielsweise in einen Hohlraum des Werkzeuges eingesetzt werden kann. Bei dem Werkzeug kann es sich um ein elektrisch antreibbares chirurgisches Werkzeug wie etwa einen chirurgischen Schraubendreher handeln. Ein beispielhafter chirurgischer Schraubendreher mit einem Aufnahmefach für eine Batterie oder eine andere elektrische Energiequelle ist in der europäischen Patentschrift EP3005963 offenbart.

Da es sich bei der Energiequelle in aller Regel um eine Gleichstromquelle handelt, ist es wichtig, dass die Energiequelle korrekt eingesetzt und die elektrischen Pole nicht vertauscht werden. Ansonsten könnte nämlich das Werkzeug unter Umständen bleibend geschädigt werden. Ein solcher Verpolungsschutz wird auch durch die Norm EN 60601 gefordert. Massnahmen zur Verhinderung einer Verpolung an sich sind bereits aus dem Stand der Technik bekannt:
Die DE 412 666 C beschreibt Polverbindungen für Batterien. Diese weisen Grundkörper und sich davon erstreckende Anschlusskontakte auf, die als Rohrstutzen ausgebildet sind. Diese Rohrstutzen der positiven und negativen Pole können verschieden grosse Durchmesser aufweisen. Die Rohrstutzen sind an ein und demselben Ende der Batterie angeordnet. Aufgrund dieser Anordnung der Pole müssen die Batterien jedoch senkrecht zu ihrer Längsachse eine vergleichsweise grosse Breite aufweisen. Der Hohlraum des Werkzeuges und entsprechend auch das Werkzeug müssen also entsprechend dimensioniert werden, damit die Batterie aufgenommen werden kann. Dies ist jedoch in vielen Fällen nachteilig, beispielweise wenn das Werkzeug ein chirurgischer Schraubendreher ist, der einhändig gehalten werden soll. Zudem wird eine Reihenschaltung mehrerer Batterien durch diese Anordnung der Pole erheblich erschwert.

Gemäss DE 2 237 279 A1 soll ein sicherer Wechselvorgang dadurch erreicht werden, dass die beiden Batteriepole als übereinanderliegende Zapfen mit verschieden grossen Durchmessern ausgebildet sind. Auch hier ist jedoch der Raumbedarf senkrecht zur Längsachse der Batterie vergleichsweise gross, und auch hier wird eine Reihenschaltung mehrerer Batterien erheblich erschwert.

Weiterhin offenbart die DE 10 2009 001 611 A1 ein Batteriefach für einen externen Herzschrittmacher. In einem Ausführungsbeispiel wird ein 9V-Block mit zwei Batteriepolen verschiedener Durchmesser verwendet. Hier ist der Raumbedarf senkrecht zur Längsachse der Batterie ebenfalls vergleichsweise gross, und eine Reihenschaltung mehrerer Batterien ist erheblich erschwert.

Die DE 36 41 927 A1 befasst sich mit einem Verpolungsschutz für stabförmige Batterien. Zu diesem Zweck ist ein in den Aufnahmeraum der Batteriehalterung hineinragender stufenförmiger Absatz vorgesehen. Die Batterien selbst enthalten nur einen einzigen sich davon erstreckenden Pol. Dies ermöglicht jedoch in vielen Fällen nur einen unzureichenden Verpolungsschutz, da nur die Dimensionen dieses einen Poles (also beispielweise dessen Durchmesser und axiale Länge) bestimmen können, ob die Batterie in zwei entgegengesetzten Orientierungen eingesetzt werden kann.

Eine weitere wichtige Anforderung, die sich insbesondere bei chirurgischen Werkzeugen ergibt, ist die Verhinderung der Wiederverwendung der Energiequelle. Um das Werkzeug nach einer Operation sterilisieren zu können, muss die Energiequelle zuvor aus diesem entnommen werden, da sie ansonsten aufgrund der aggressiven Sterilisationsbedingungen zerstört werden könnte, beispielsweise indem sie auslaufen könnte, wodurch auch der Sterilisator verunreinigt werden könnte. Übliche Energiequellen lassen sich ohne weiteres nach der Sterilisation ein zweites Mal in das Werkzeug einsetzen. Auf diese Weise könnte das Werkzeug mit einer bereits benutzten und daher zumindest teilweise entladenen Energiequelle verwendet werden. Dieses Risiko ist besonders dann gegeben, wenn der Energiequelle äusserlich nicht angesehen werden kann, ob sie bereits zuvor in einem Werkzeug verwendet wurde. Wird dies erst während der nächsten Operation bemerkt, so kann dies unter Umständen zu Verzögerungen und damit auch zu nachteiligen Konsequenzen für den Patienten führen. Denn insbesondere kann es zu einem intraoperativen Ausfall des Werkzeuges kommen. Auch um die "wesentlichen Leistungsmerkmale" gemäss der bereits erwähnten Norm EN 60601 erreichen zu können, muss die Wiederverwendung der Energiequelle verhindert werden.

Die US 2006/0117911 A1 offenbart batteriebetriebene chirurgische Schraubendreher, die zur einmaligen Verwendung vorgesehen sind. Spezielle bauliche Massnahmen, die die Wiederverwendung der Batterie verhindern, werden allerdings nicht offenbart.

Die US 6,126,670 befasst sich mit Einwegbatteriepacks, die mit einem chirurgischen Werkzeug verbunden werden können, um dieses anzutreiben. Die korrekte Polung wird mit Hilfe eines am Werkzeug angeordneten Zapfens erreicht, welcher in eine korrespondierende Öffnung des Batteriepacks eingreift. Dieser Mechanismus verhindert allerdings nicht wirkungsvoll die Wiederverwendbarkeit des Batteriepacks.

Die DE 20 2009 017 971 U1 befasst sich mit Handgriffen für chirurgische Instrumente. Im Handgriff ist ein Akkumulator angeordnet, der nach Entfernen eines Deckels aus dem Handgriff entnehmbar ist. Auch dieses Dokument offenbart jedoch keinen Mechanismus, der das Wiedereinsetzen des Akkumulators verhindert. Im Gegenteil wird sogar ausgeführt, dass der Akkumulator nach Sterilisation des Hohlkörpers wieder in diesen eingesetzt werden kann.

Gegenstand der US 4,309,067 sind Schnittstellen für das mechanische und elektrische Verbinden eines Batteriepacks mit einem Werkzeug, die eine möglichst einhändige Entfernung des Batteriepacks erlauben sollen. Diese Verbindung erfolgt mit Hilfe eines Verschlusses, der über einen Auslöseknopf betätigt werden kann, welcher sich durch einen Schlitz erstreckt. Auch in diesem Dokument ist allerdings kein Mechanismus offenbart, der das wiederholte Einsetzen des Batteriepacks verhindert.

Das Dokument US 6,257,351 offenbart ebenfalls einen Batteriepack, der mit einem chirurgischen Instrument lösbar verbindbar ist. Aber auch hier ist kein Mechanismus offenbart, der ein Wiedereinsetzen des Batteriepacks verhindert.

Die EP 1 813 200 A2 offenbart chirurgische Instrumente mit entfernbarer Batterie. In einer Ausführungsform enthält das Instrument einen primären Abschnitt und einen Griffabschnitt, welcher lösbar mit dem primären Abschnitt verbindbar ist und eine Batterie enthält. Der Griffabschnitt verfügt über einen Abbruchabschnitt, der nach dem Trennen des Griffabschnittes vom primären Abschnitt an diesem verbleibt, so dass ein weiteres Zusammensetzten der genannten Teile nicht mehr möglich ist. Die Wiederverwendung der Batterie selbst wird dadurch aber nicht verhindert.

Zudem ist ein weiteres Ausführungsbeispiel eines Instrumentes offenbart, welches über einen Zähler und eine Motorsperre verfügt. In einer ersten Variante dieses weiteren Ausführungsbeispiels ist der Zähler derart ausgebildet und angeordnet, dass die Motorsperre aktiviert wird, nachdem die Batterie eine vorbestimmt Anzahl von Malen (beispielsweise zweimal) vom Instrument entfernt wurde. Eine zweite Variante sieht vor, dass die Motorsperre aktiviert wird, nachdem der Griffabschnitt eine vorbestimmt Anzahl von Malen (beispielsweise zweimal) vom primären Abschnitt getrennt wurde. Beide Varianten sind jedoch baulich aufwändig und zudem störungsanfällig.

Es ist daher eine Aufgabe der vorliegenden Erfindung, eine elektrische Energiequelle für ein elektrisch antreibbares Werkzeug bereitzustellen, mit der mindestens einer der oben beschriebenen Nachteile des Standes der Technik überwunden werden können. Insbesondere soll die Energiequelle zumindest in einigen Ausführungsformen in einer senkrecht zur Längsachse eines Grundkörpers der Energiequelle verlaufenden Richtung eine möglichst geringe Baugrösse aufweisen, ohne dass auf den im Stand der Technik an sich bekannten Verpolungsschutz verzichtet werden muss. Weiterhin soll insbesondere zumindest in einigen Ausführungsformen auf baulich möglichst einfache Weise eine Wiederverwendung einer bereits zuvor in einem Werkzeug eingesetzten Energiequelle verhindert werden.

Obige Aufgaben werden gelöst durch eine elektrische Energiequelle für ein elektrisch antreibbares Werkzeug, welche enthält:
- ein Gehäuse, welches insbesondere zumindest teilweise einen wie oben beschriebenen Grundkörper bildet, insbesondere die Aussenseite des Grundkörpers;
- mindestens eine im Gehäuse angeordnete Spannungsquelle zum Erzeugen einer elektrischen Spannung;
- mindestens einen zweiten Pol, insbesondere einen wie oben beschriebenen zweiten Pol;
- mindestens einen inneren elektrischen Kontakt,
wobei die von der Spannungsquelle erzeugte Spannung zwischen dem zweiten Pol und dem inneren elektrischen Kontakt anliegt oder anlegbar ist. Die Energiequelle kann eine oder mehrere Spannungsquellen enthalten, die parallel oder in Reihe geschaltet sein können.

Gemäss einem erfindungsgemässen Aspekt enthält die Energiequelle mindestens ein elektrisches Kontaktelement mit einem ersten elektrischen Kontakt und einem zweiten elektrischen Kontakt, welche elektrisch miteinander verbunden sind, wobei
- das Kontaktelement einen ersten Pol enthält, der den zweiten Kontakt enthält;
- sich das Kontaktelement in einer Bereitschaftsposition befindet, in der es lösbar direkt oder indirekt mit dem Gehäuse verbunden ist und in der weder der erste Kontakt noch der zweite Kontakt in elektrischer Verbindung mit dem inneren Kontakt steht;
- das Kontaktelement durch Einwirkung einer Kraft, welche durch ein erstes Abgreifelement des Werkzeuges ausübbar ist, von der Bereitschaftsposition in eine Betriebsposition bewegbar ist, in der der erste Kontakt in elektrischer Verbindung mit dem inneren Kontakt steht, so dass die von der Spannungsquelle erzeugte Spannung zwischen dem zweiten Pol und dem ersten Abgreifelement anliegt;
- sich das Kontaktelement nach erstmaligem Entfernen des ersten Abgreifelements aus der Betriebsposition in eine Endposition bewegt, in der das Kontaktelement nicht mehr in elektrischen Kontakt mit dem ersten Abgreifelement bringbar ist, so dass die von der Spannungsquelle erzeugte Spannung nicht mehr zwischen dem zweiten Pol und dem ersten Abgreifelement anlegbar ist.

Das Kontaktelement kann insbesondere einstückig und aus einem elektrisch leitfähigen Material gebildet sein, wie beispielsweise aus Messing, insbesondere aus vergoldetem oder vernickeltem Messing.

Es ist denkbar, dass die Energiequelle enthält
- einen Grundkörper, welcher sich entlang einer Längsachse erstreckt, mit einem ersten Ende und einem dem ersten Ende bezüglich der Längsachse gegenüberliegenden zweiten Ende,
- mindestens einen ersten elektrischen Pol mit einem ersten Durchmesser sowie
- mindestens einen zweiten elektrischen Pol mit einem zweiten Durchmesser.

Dabei liegt zwischen dem ersten Pol und dem zweiten Pol eine elektrische Spannung an, oder eine solche elektrische Spannung ist zwischen diesen beiden Polen anlegbar. Der erste Durchmesser ist vom zweiten Durchmesser verschieden. Insbesondere kann der erste Durchmesser kleiner sein als der zweite Durchmesser.

Unter einem "Durchmesser" eines Poles wird hier und im Folgenden das Doppelte des maximalen Abstands aller Punkte dieses Poles von der Längsachse des Grundkörpers verstanden. Mit anderen Worten ist der Durchmesser des Poles der kleinste Innendurchmesser eines imaginären Hohlzylinders mit kreiszylinderförmigem Hohlraum, dessen Längsachse mit der Längsachse des Grundkörpers übereinstimmt und in den der Pol vollständig aufnehmbar ist.

In der baulich einfachsten Ausgestaltung können einer oder beide der Pole kreiszylinderförmig ausgestaltet sein, wobei die Längsachsen dieser Kreiszylinder parallel zur Längsachse des Grundkörpers verlaufen können oder sogar damit übereinstimmen können. Alternativ können einer oder beide Pole aber auch bezüglich der Längsachse des Grundkörpers exzentrisch angeordnet sein und/oder beispielsweise kegelförmig oder kegelstumpfförmig ausgebildet sein; hierzu wird auf die weiter unten noch im Detail beschriebenen Ausführungsbeispiele verwiesen.

Bevorzugt erstreckt sich der erste elektrische Pol vom ersten Ende des Grundkörpers weg, und der zweite elektrische Pol erstreckt sich vom zweiten Ende des Grundkörpers weg.

Bei entsprechend geeigneter Ausbildung des elektrisch antreibbaren Werkzeuges kann eine Verpolung verhindert werden, wie weiter unten ebenfalls noch detailliert erläutert wird. Da sich die beiden Pole von gegenüberliegenden Enden des Grundkörpers weg erstrecken, wird in einer senkrecht zur Längsachse des Grundkörpers verlaufenden Richtung eine sehr geringe Baugrösse der Energiequelle ermöglicht. Auf diese Weise kann die Energiequelle beispielsweise in einen elektrischen Schraubendreher mit vergleichsweise geringem Durchmesser eingesetzt werden. Weiterhin können durch die Anordnung der Pole an gegenüberliegenden Enden des Grundkörpers mehrere Energiequellen einfacher in Reihe geschaltet werden. Da sich zwei Pole vom Grundkörper weg erstrecken und diese verschiedene Durchmesser haben, ergeben sich zudem gegenüber Energiequellen mit nur einem einzigen sich vom Grundkörper weg erstreckenden Pol weitere Dimensionen (also beispielsweise zwei Durchmesser und zwei axiale Längen), die darüber bestimmen können, ob die Energiequelle in zwei entgegengesetzten Orientierungen eingesetzt werden kann. Somit kann die Energiequelle noch sicherer gegen eine Verpolung geschützt werden, indem die genannten Dimensionen auf die entsprechenden Dimensionen des Werkzeuges abgestimmt werden.

Der Durchmesser der Energiequelle kann im Bereich von 3 mm bis 50 mm liegen. Unter dem Durchmesser der Energiequelle wird analog zur obigen Definition das Doppelte des maximalen Abstands aller Punkte der Energiequelle von der Längsachse des Grundkörpers verstanden. D.h. ausser Energiequellen mit zylindrischem Querschnitt sind beispielsweise auch solche mit quadratischem oder rechteckigem Querschnitt. Ferner kann das Verhältnis des Durchmessers des ersten Poles und/oder des zweiten Poles zum Durchmesser der Energiequelle grösser sein als 50 %. Bei vorgegebenem Durchmesser der Energiequelle (der beispielsweise durch die Dimensionen des Werkzeuges beschränkt sein kann) kann der Durchmesser des ersten und/oder zweiten Poles also vergleichsweise gross gewählt werden, um eine grössere elektrische Kontaktfläche herzustellen. Bei einer Anordnung beider Pole an ein und demselben Ende des Grundkörpers wie im Stand der Technik wäre die Kontaktfläche kleiner. Umgekehrt kann das Verhältnis des Durchmessers des ersten Poles und/oder des zweiten Poles zum Durchmesser der Energiequelle kleiner sein als 50 %.

Das Verhältnis des ersten Durchmessers des ersten Poles zum zweiten Durchmesser des zweiten Poles kann im Bereich von 1 % bis 99 %, bevorzugt von 70 % bis 96 %, besonders bevorzugt von 80 % bis 86 % liegen. Der erste Durchmesser des ersten Poles kann im Bereich von 1 mm bis 49 mm, bevorzugt von 2 mm bis 3 mm, besonders bevorzugt von 2,4 mm bis 2,6 mm liegen. Der zweite Durchmesser des zweiten Poles kann im Bereich von 1 mm bis 49 mm, bevorzugt von 2,5 mm bis 3,5 mm, besonders bevorzugt von 2,9 mm bis 3,1 mm liegen. Der erste und/oder der zweite Pol kann entlang der Längsachse des Grundkörpers eine Länge aufweisen, die im Bereich von 0,05 mm bis 20 mm, bevorzugt von 0,1 mm bis 1 mm, besonders bevorzugt von 0,15 mm bis 0,25 mm liegt.

Der erste und/oder der zweite Pol kann in einigen Ausführungsformen starr mit dem Grundkörper verbunden sein. Es ist jedoch auch denkbar, dass mindestens einer der Pole gegenüber dem Grundkörper federnd gelagert oder als Feder ausgeführt ist. Dies kann ein einfacheres Einsetzen der Energiequelle in das Werkzeug ermöglichen sowie für eine (insbesondere zusätzliche) Klemmwirkung sorgen, die den sicheren mechanischen und damit elektrischen Kontakt zwischen den Polen der Energiequelle und Abgreifelementen des Werkzeuges gewährleistet.

Bevorzugt ist der Grundkörper im Wesentlichen kreiszylinderförmig ausgebildet. Dies hat den Vorteil, dass die Energiequelle unabhängig von ihrer Drehposition um die Längsachse herum vom Werkzeug aufgenommen werden kann - jedenfalls dann, wenn die beiden Pole und die damit in Kontakt zu bringenden Abgreifelemente des Werkzeuges dies ebenfalls erlauben.

In der anfänglichen Bereitschaftsposition ist das Kontaktelement also lösbar mit dem Gehäuse verbunden. Beispielsweise kann es in der Bereitschaftsposition am Gehäuse eingeklemmt, eingeklebt, eingepresst oder eingeschweisst sein oder über mindestens eine Sollbruchstelle mit dem Gehäuse verbunden sein. In dieser Bereitschaftsposition ist weder der erste Kontakt noch der zweite Kontakt des Kontaktelementes in elektrischer Verbindung mit dem inneren Kontakt. Infolgedessen liegt auch zwischen den Kontakten des Kontaktelementes und dem zweiten Pol der Energiequelle keine Spannung an.

Durch Einwirkung einer Kraft, welche durch das erste Abgreifelement ausübbar ist, kann das Kontaktelement von der Bereitschaftsposition in die Betriebsposition bewegt werden. Dort steht der erste Kontakt in elektrischer Verbindung mit dem inneren Kontakt. Auf diese Weise liegt die von der Spannungsquelle erzeugte Spannung zwischen dem zweiten Pol und dem ersten Abgreifelement an. Ist dabei auch der zweite Pol in Kontakt mit einem zweiten Abgreifelement des Werkzeuges, so liegt diese Spannung zwischen den beiden Abgreifelementen an, so dass das elektrische Werkzeug angetrieben werden kann.

Nach erstmaligem Entfernen des ersten Abgreifelementes bewegt sich das Kontaktelement aus der Betriebsposition in die Endposition. Die Kraft für diese Bewegung kann beispielsweise durch die Schwerkraft oder durch eine Feder erzeugt werden. In der Endposition ist das Kontaktelement nicht mehr ohne weiteres in elektrischen Kontakt mit dem ersten Abgreifelement bringbar. Folglich ist auch die von der Spannungsquelle erzeugte Spannung nicht mehr zwischen dem zweiten Pol und dem ersten Abgreifelement anlegbar. Mit anderen Worten ist das Kontaktelement nach erstmaligem Entfernen des ersten Abgreifelementes nicht mehr ohne weiteres in eine Position bewegbar, in der die Energiequelle nochmals zum Antreiben des Werkzeuges geeignet ist. Somit ist eine Wiederverwendung der Energiequelle wirksam ausgeschlossen, insbesondere die Wiederverwendung einer bereits benutzten und daher zumindest teilweise entladenen Energiequelle. Zudem kann der Energiequelle in vielen Ausführungsbeispielen aufgrund der veränderten Position des Kontaktelementes äusserlich angesehen werden, dass sie bereits in einem Werkzeug verwendet wurde.

Insbesondere wenn das Kontaktelement in der Bereitschaftsposition am Gehäuse eingepresst ist, kann das Kontaktelement eine Haltefläche aufweisen, und das Gehäuse kann eine Gegenhaltefläche aufweisen, wobei das Kontaktelement in der Bereitschaftsposition durch mechanischen Kontakt der Haltefläche mit der Gegenhaltefläche haltbar ist. Alternativ dazu ist es aber beispielsweise auch möglich, dass das Kontaktelement in der Bereitschaftsposition durch magnetische Kräfte am Gehäuse gehalten ist.

Es ist denkbar und liegt im Rahmen der Erfindung, dass nicht nur der erste Pol, sondern auch der zweite Pol der Energiequelle Teil eines wie oben beschriebenen elektrischen Kontaktelementes ist. Genauer kann die Energiequelle einen zweiten inneren elektrischen Kontakt und ein zweites elektrisches Kontaktelement mit einem ersten elektrischen Kontakt und einem zweiten elektrischen Kontakt aufweisen, welche miteinander verbunden sind, wobei
- das zweite Kontaktelement den zweiten Pol enthält, der den zweiten Kontakt enthält;
- sich das zweite Kontaktelement in einer Bereitschaftsposition befindet, in der es lösbar direkt oder indirekt mit dem Gehäuse verbunden ist und in der weder der erste Kontakt noch der zweite Kontakt des zweiten Kontaktelements in elektrischer Verbindung mit dem zweiten inneren Kontakt steht;
- das zweite Kontaktelement durch Einwirkung einer Kraft, welche durch ein zweites Abgreifelement des Werkzeuges ausübbar ist, von der Bereitschaftsposition in eine Betriebsposition bewegbar ist, in der der erste Kontakt des zweiten Kontaktelements in elektrischer Verbindung mit dem zweiten inneren Kontakt steht, so dass die von der Spannungsquelle erzeugte Spannung zwischen dem ersten Pol und dem zweiten Abgreifelement anliegt;
- sich das zweite Kontaktelement nach erstmaligem Entfernen des zweiten Abgreifelements aus der Betriebsposition in eine Endposition bewegt, in der das zweite Kontaktelement nicht mehr in elektrischen Kontakt mit dem zweiten Abgreifelement bringbar ist, so dass die von der Spannungsquelle erzeugte Spannung nicht mehr zwischen dem ersten Pol und dem zweiten Abgreifelement anlegbar ist.

Das Kontaktelement kann einen Haltekörper aufweisen, an dem die Haltefläche angeordnet ist. Dieser Haltekörper kann im Wesentlichen kreiszylinderförmig ausgebildet sein und eine im Wesentlichen kreiszylindermantelförmige Haltefläche aufweisen. Die Gegenhaltefläche kann ebenfalls im Wesentlichen kreiszylindermantelförmig ausgebildet sein, wobei natürlich auch andere Formen denkbar sind und im Rahmen der Erfindung liegen. Dies ermöglicht es, während der Herstellung der Energiequelle das Kontaktelement unabhängig von der Drehposition um die Zylinderachse herum mit dem Gehäuse zu verbinden, um die anfängliche Bereitschaftsposition zu erhalten.

Vorteilhafterweise befindet sich der Haltekörper zumindest in der Bereitschaftsposition auf der dem inneren Kontakt zugewandten Seite des Kontaktelementes, und der erste Pol befindet sich zumindest in der Bereitschaftsposition auf der dem inneren Kontakt abgewandten Seite des Kontaktelementes.

Der Haltekörper des Kontaktelementes kann einen Haltekörperdurchmesser aufweisen, der grösser ist als der erste Durchmesser des ersten Poles. Hierdurch kann das Kontaktelement nur durch mechanischen Kontakt zwischen Kontaktelement und Gehäuse an letzterem gehalten werden, nicht aber durch mechanischen Kontakt zwischen erstem Pol und Gehäuse.

Das Kontaktelement kann entlang einer Längsachse der Haltefläche von der Bereitschaftsposition in die Betriebsposition bewegbar sein. Bei der Längsachse kann es sich beispielsweise um eine Symmetrieachse der Haltefläche handeln. Bevorzugt stimmt die Längsachse der Haltefläche mit der Längsachse des Grundkörpers der Energiequelle überein. Auf diese Weise kann das Kontaktelement besonders einfach von der Bereitschaftsposition in die Betriebsposition bewegt werden, wenn das erste Abgreifelement des Werkzeuges eine Kraft darauf ausübt.

Der erste Kontakt des Kontaktelementes kann an einer Deckelfläche des ersten Poles ausgebildet sein, insbesondere an einer Deckelfläche eines kreiszylinderförmig ausgebildeten ersten Poles. Auf diese Weise kann ganz besonders einfach ein elektrischer Kontakt zwischen dem ersten Pol und dem ersten Abgreifelement des Werkzeuges hergestellt werden.

Die Energiequelle und insbesondere ihr Kontaktelement können derart ausgebildet sein, dass das Kontaktelement in der Betriebsposition zwischen dem inneren Kontakt und dem ersten Abgreifelement eingeklemmt ist. Insbesondere kann diese Klemmung in Richtung der Längsachse des Grundkörpers erfolgen. Das Einklemmen kann verhindern, dass das Kontaktelement in der Betriebsposition verrutscht.

Bevorzugt enthält die Energiequelle mindestens einen Hohlraum, in dem das Kontaktelement in der Endposition lose angeordnet ist. Diese lose Anordnung des Kontaktelementes in der Endposition bewirkt, dass das Kontaktelement innerhalb des Hohlraumes verrutschen kann, insbesondere in einer Ebene senkrecht zur Längsachse des Gehäuses. Das Kontaktelement kann also nicht mehr ohne weiteres in die Betriebsposition überführt werden, in der das erste Abgreifelement in elektrischen Kontakt mit dem ersten Kontakt des Kontaktelementes bringbar ist.

Um diese lose Anordnung zu bewirken, ist es vorteilhaft, wenn der Hohlraum entlang der Längsachse über eine Ausdehnung verfügt, die grösser ist als die entlang der Längsachse des Grundkörpers gemessene Höhe des Haltekörpers des Kontaktelementes.

Ebenfalls bevorzugt ist es zu diesem Zweck, wenn der Hohlraum senkrecht zur Längsachse des Grundkörpers einen Hohlraumdurchmesser aufweist, der grösser ist als der Haltekörperdurchmesser. Auch hier wird der Durchmesser des Hohlraumes analog zur obigen Definition verstanden. Der Hohlraum kann im Wesentlichen kreiszylinderförmig ausgebildet sein, wobei die Symmetrieachse mit der Längsachse des Grundkörpers übereinstimmt.

Alternativ oder zusätzlich kann die Rückkehr des Kontaktelementes in die Betriebsposition nach erstmaligem Entfernen des ersten Abgreifelementes auch dadurch verhindert werden, dass das Kontaktelement durch weitere Kräfte von der Betriebsposition ferngehalten wird. Dies kann beispielsweise mit Hilfe mindestens eines Magnetes und/oder mittels mindestens einer Feder erreicht werden.

Zur Aufnahme der Energiequelle kann das Werkzeug einen Hohlraum aufweisen. Vorteilhafterweise ist das erste Abgreifelement an der Innenseite eines Deckels des Werkzeuges angeordnet. Dabei ist dieser Deckel zum Halten der Energiequelle innerhalb des genannten und optional auch zum Verschliessen dieses Hohlraumes ausgebildet. Diese Ausgestaltung erlaubt es, dass durch Aufsetzen des Deckels sowohl das erste Abgreifelement eine Kraft auf das Kontaktelement ausübt, um dieses von der Bereitschaftsposition in die Betriebsposition zu bewegen, als auch die Energiequelle insgesamt innerhalb des Hohlraumes gehalten wird und optional der Hohlraum verschlossen wird, so dass ein sicherer mechanischer und damit elektrischer Kontakt auch am zweiten Pol hergestellt wird.

Ein weiterer Aspekt der vorliegenden Erfindung betrifft ein Werkzeugset. Dieses Werkzeugset enthält
- mindestens ein elektrisch antreibbares Werkzeug, insbesondere mindestens ein elektrisch antreibbares chirurgisches Werkzeug, insbesondere mindestens einen elektrisch antreibbaren chirurgischen Schraubendreher,
- mindestens eine wie oben beschriebene elektrische Energiequelle, mittels deren das Werkzeug elektrisch antreibbar ist.

Insbesondere kann das Werkzeug ein erstes Abgreifelement aufweisen, welches derart ausgebildet ist und angeordnet ist, dass das Kontaktelement der Energiequelle durch Einwirkung einer Kraft, welche durch das erste Abgreifelement ausübbar ist, von der Bereitschaftsposition in die Betriebsposition bewegbar ist. Wie oben bereits erläutert wurde, kann das Werkzeug einen Hohlraum zum Aufnehmen der Energiequelle aufweisen. Ferner kann das erste Abgreifelement an der Innenseite eines Deckels des Werkzeuges angeordnet sein, wobei dieser Deckel zum Halten der Energiequelle innerhalb des Hohlraumes optional zum Verschliessen dieses Hohlraumes ausgebildet sein kann.

Wenn die Energiequelle zwei elektrische Pole mit verschiedenen Durchmessern aufweist und diese Pole sich von gegenüberliegenden Enden eines Grundkörpers der Energiequelle weg erstrecken, so weist das Werkzeug bevorzugt ein erstes Abgreifelement und ein zweites Abgreifelement zum Abgreifen der zwischen dem ersten Pol und dem zweiten Pol anliegenden oder anlegbaren elektrischen Spannung auf. Dabei sind die Energiequelle und das Werkzeug derart ausgebildet und aufeinander abgestimmt, dass
- gleichzeitig der erste elektrische Pol mit dem ersten Abgreifelement in Kontakt bringbar ist und der zweite elektrische Pol mit dem zweiten Abgreifelement in Kontakt bringbar ist und
- der erste elektrische Pol nicht mit dem zweiten Abgreifelement in Kontakt bringbar ist
   und/oder
   der zweite elektrische Pol nicht mit dem ersten Abgreifelement in Kontakt bringbar ist.

Folglich kann die zwischen den beiden Polen der Energiequelle anliegende oder anlegbare elektrische Spannung nur in einer einzigen Polung an die beiden Abgreifelemente übergeben werden; eine umgekehrte Polung ist nicht möglich.

Um dies zu bewerkstelligen, kann das Werkzeug, insbesondere ein Deckel des Werkzeuges, einen ersten elektrischen Isolator enthalten, welcher das erste Abgreifelement ringförmig umschliesst und in Längsrichtung des Werkzeuges überragt. Somit entsteht ein teilweise begrenzter erster Hohlraum, der durch eine Innenwand des ersten Isolators und das erste Abgreifelement begrenzt ist. Der erste Hohlraum ist so bemessen, dass nur der erste Pol der Energiequelle im ersten Hohlraum aufnehmbar und in Kontakt mit dem ersten Abgreifelement bringbar ist, nicht aber auch der zweite Pol. Insbesondere kann der erste Hohlraum eine in Längsrichtung des Werkzeuges gemessene erste Tiefe aufweisen, die kleiner ist als die erste Höhe des ersten Poles, aber grösser als die erste Höhe des zweiten Poles. Alternativ oder zusätzlich kann der erste Hohlraum einen Innendurchmesser aufweisen, der grösser ist als der erste Durchmesser des ersten Poles, aber kleiner als der zweite Durchmesser des zweiten Poles.

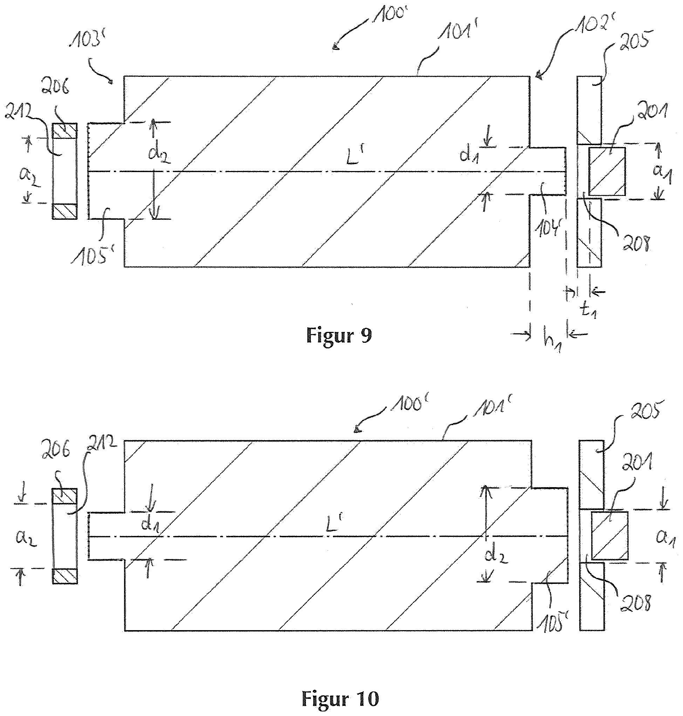

Ferner enthält das zweite Abgreifelement bevorzugt eine ringförmige zweite Abgreiffläche, die dem Hohlraum des Werkzeuges zugewandt sein kann. Das zweite Abgreifelement kann einen zweiten Isolator ringförmig umschliessen und diesen in Längsrichtung des Werkzeuges überragen. Auf diese Weise kann ein zweiter Hohlraum gebildet werden, der durch eine Innenwand des zweiten Abgreifelementes und den zweiten Isolator begrenzt ist. Der Innendurchmesser der zweiten Abgreiffläche ist bevorzugt kleiner als der zweite Durchmesser des zweiten Poles der Energiequelle, aber grösser als der erste Durchmesser des ersten Poles der Energiequelle. Hierdurch kann nur der zweite Pol in Kontakt mit dem zweiten Abgreifelement gebracht werden. Der erste Pol hingegen dringt in den zweiten Hohlraum hinein, ohne in Kontakt mit dem zweiten Abgreifelement gelangen zu können. Aus den im Zusammenhang mit den Figuren 9 und 10 erläuterten Gründen ist es ebenfalls vorteilhaft, wenn der Innendurchmesser des ersten Hohlraums kleiner ist als der Innendurchmesser der zweiten Abgreiffläche.

Mit weiterem Vorteil ist die in Längsrichtung des Werkzeuges gemessene zweite Tiefe des zweiten Hohlraumes kleiner als die entlang der Längsachse der Energiequelle gemessene erste Länge des ersten Poles. Dies ist insbesondere dann vorteilhaft, wenn die Energiequelle ein wie oben beschriebenes bewegliches Kontaktelement enthält, welches den Haltekörper und den ersten Pol einstückig enthält und insgesamt aus einem leitfähigen Material gebildet ist und der Durchmesser des Haltekörpers grösser ist als der Innendurchmesser des zweiten Abgreifelementes. Denn dann stösst der erste Pol am zweiten Isolator an, bevor der Haltekörper in Kontakt mit der zweiten Abgreiffläche gelangen kann.

Alternativ oder zusätzlich kann die in Längsrichtung des Werkzeuges gemessene zweite Tiefe des zweiten Hohlraumes kleiner sein als die entlang der Längsachse der Energiequelle gemessene zweite Länge des zweiten Poles, aber grösser als die entlang der Längsachse der Energiequelle gemessene erste Länge des ersten Poles. In diesem Falle sollte jedoch die Deckelfläche am ersten Ende der Energiequelle, von der sich der erste Pol weg erstreckt, als Isolator ausgebildet sein.

Von Vorteil kann es ebenfalls sein, wenn das zweite Abgreifelement derart innerhalb eines Ringisolators mit einer ringförmigen Isolatorfläche angeordnet ist, dass die zweite Abgreiffläche gegenüber der Isolatorfläche um einen Abstand versenkt ist, der kleiner als die Länge des zweiten Poles ist. Denn hierdurch kann verhindert werden, dass eine Energiequelle mit einem Pol, der sich jedoch nicht vom Grundkörper weg erstrecket, derart einsetzbar ist, dass dieser Pol in Kontakt mit der zweiten Abgreiffläche gerät. Dieser Vorteil entsteht auch dann, wenn die Länge des Poles kleiner ist als der genannte Abstand.

Noch ein weiterer Aspekt der Erfindung betrifft ein Verfahren zum Einsetzen einer wie oben beschriebenen Energiequelle mit elektrischem Kontaktelement in ein elektrisch antreibbares Werkzeug. Bei diesem Werkzeug kann es sich wiederum um ein elektrisch antreibbares chirurgisches Werkzeug handeln, insbesondere um einen elektrisch antreibbaren chirurgischen Schraubendreher. Das Verfahren enthält einen Schritt, in dem das Kontaktelement der Energiequelle durch Einwirkung einer Kraft durch das erste Abgreifelement des Werkzeuges von der Bereitschaftsposition in die Betriebsposition bewegt wird. Wie sich aus den obigen Ausführungen ergibt, wird hierdurch erreicht, dass die von der Spannungsquelle erzeugte Spannung zwischen dem zweiten Pol der Energiequelle und dem ersten Abgreifelement anliegt, wodurch das Werkzeug angetrieben werden kann.

Im Folgenden wird die Erfindung anhand von mehreren Ausführungsbeispielen und Zeichnungen näher erläutert. Dabei zeigen
- Figur 1:: eine seitliche Schnittansicht einer ersten erfindungsgemässen Energiequelle;
- Figur 2:: eine seitliche Schnittansicht der in einem elektrischen Schraubendreher in richtiger Polung aufgenommenen ersten erfindungsgemässen Energiequelle;
- Figur 3:: eine Detailansicht auf einen zweiten Pol der ersten erfindungsgemässen Energiequelle und ein zweites Abgreifelement des Schraubendrehers;
- Figur 4:: eine Detailansicht auf einen ersten Pol der ersten erfindungsgemässen Energiequelle und ein erstes Abgreifelement des Schraubendrehers;
- Figur 5:: eine Detailansicht auf den ersten Pol der ersten erfindungsgemässen Energiequelle in der Bereitschaftsposition;
- Figur 6:: eine Detailansicht auf die erste erfindungsgemässe Energiequelle in der Betriebsposition;
- Figur 7:: eine Detailansicht auf den ersten Pol der ersten erfindungsgemässen Energiequelle in der Endposition;
- Figur 8:: eine seitliche Schnittansicht auf die in falscher Polung eingesetzte erste erfindungsgemässe Energiequelle;
- Figur 9:: eine schematische Seitenansicht auf eine Energiequelle und zwei Abgreifelemente in korrekter Polung;
- Figur 10:: eine schematische Seitenansicht auf eine Energiequelle und zwei Abgreifelemente in falscher Polung;
- Figuren 11a-i:: schematische perspektivische Ansichten auf weitere Energiequellen.

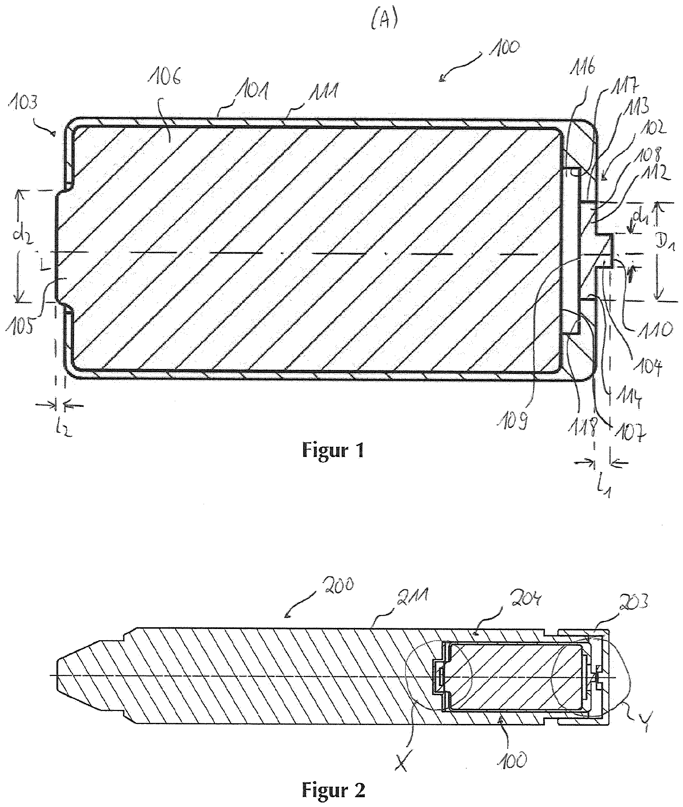

Die in Figur 1 dargestellte elektrische Energiequelle 100 enthält einen Grundkörper 101, welcher sich entlang einer Längsachse L erstreckt. Der Grundkörper 101 verfügt über ein erstes Ende 102, von dem sich ein erster elektrischer Pol 104 weg erstreckt, sowie ein dem ersten Ende 102 bezüglich der Längsachse L gegenüberliegendes zweites Ende 103, von dem sich ein zweiter elektrischer Pol 105 weg erstreckt. Der Grundkörper 101 hat eine Länge von 41,6 mm und einen Durchmesser von 15 mm. Der erste elektrische Pol 104 weist einen ersten Durchmesser d₁ = 2,5 mm und eine entlang der Längsachse L der Energiequelle 100 gemessene erste Länge l₁ = 0,2 mm auf, und der zweite elektrische Pol 105 weist einen zweiten Durchmesser d₂ = 3 mm auf und eine entlang der Längsachse L der Energiequelle 100 gemessene zweite Länge l₂ = 0,2 mm auf. Insbesondere ist also der erste Durchmesser d₁ kleiner als der zweite Durchmesser d₂.

Die Energiequelle 100 enthält ein Gehäuse 111, welches die Aussenseite des Grundkörpers 101 bildet. Im Gehäuse 111 befindet sich eine kreiszylinderförmige Spannungsquelle 106. Dabei kann es sich beispielsweise um eine an sich bekannte Einzelzelle CR 2 oder eine andere an sich bekannte Rundzelle handeln. Die von der Spannungsquelle 106 erzeugte Spannung liegt zwischen dem zweiten Pol 105 und einem inneren elektrischen Kontakt 107 an, die sich von entgegengesetzten Enden der Spannungsquelle 106 weg erstrecken.

Die Energiequelle 100 verfügt weiterhin über ein elektrisches Kontaktelement 108. Dieses enthält einen kreiszylinderförmigen Haltekörper 112 mit einer in Umfangsrichtung gebildeten kreiszylindermantelförmigen Haltefläche 113. Am Haltekörper 112 ist der ebenfalls kreiszylinderförmige erste Pol 104 angeformt. Das Kontaktelement 108 verfügt somit über einen ersten elektrischen Kontakt 109, der durch die dem ersten Pol 104 gegenüberliegende Deckelfläche des Haltekörpers 112 gebildet ist und dem inneren Kontakt 107 zugewandt ist, sowie einen zweiten elektrischen Kontakt 110, welcher an der dem Haltekörper 112 gegenüberliegenden Deckelfläche des ersten Poles 104 gebildet ist und dem inneren Kontakt 107 abgewandt ist. Der Haltekörper 112 weist einen Haltekörperdurchmesser D₁ auf, der grösser ist als der erste Durchmesser d₁ des ersten Poles 104. Das Kontaktelement 108 ist einstückig aus einem elektrisch leitfähigen Material gebildet, wie beispielsweise aus vergoldetem oder vernickeltem Messing, so dass der erste Kontakt 109 und der zweite Kontakt 110 elektrisch miteinander verbunden sind.

Figur 1 zeigt die Energiequelle 100 in einer Bereitschaftsposition A, in der das Kontaktelement 108 am Gehäuse 111 lösbar eingeklemmt ist, wobei die Haltefläche 113 durch mechanischen Kontakt mit einer am Gehäuse 111 gebildeten kreiszylindermantelförmigen Gegenhaltefläche 114 gehalten wird. Der erste Pol 104 erstreckt sich somit in der vom inneren Kontakt 107 abgewandten Richtung weg, also vom Grundkörper 101 der Energiequelle 100 weg.

Zwischen der Spannungsquelle 106 und dem Kontaktelement 110 ist ein erster Hohlraum 116 gebildet, der teilweise vom inneren Kontakt 107 sowie von einer kreiszylindermantelförmigen Innenwand 117 und einer kreisringförmigen Innenwand 118 des Gehäuses 111 begrenzt ist. In der Bereitschaftsposition A ist der erste Hohlraum 116 zudem an der dem inneren Kontakt 107 gegenüberliegenden Seite des ersten Kontaktes 109 begrenzt. In der Bereitschaftsposition A dringt das Kontaktelement 108 aber nicht in den ersten Hohlraum 116 ein. Es wäre aber auch möglich, dass das Kontaktelement 108 in der Bereitschaftsposition A teilweise in den ersten Hohlraum 116 eindringt, ohne dass jedoch ein Kontakt zwischen dem inneren Kontakt 107 und dem ersten Kontakt 109 besteht.

Aufgrund des fehlenden elektrischen Kontaktes zwischen dem inneren Kontakt 107 und dem ersten Kontakt 109 des Kontaktelements 108 liegt in der Bereitschaftsposition A zwischen dem ersten Pol 104 und dem zweiten Pol 105 keine elektrische Spannung an.

Figur 2 zeigt schematisch einen elektrisch antreibbaren chirurgischen Schraubendreher 200. Dieser verfügt über ein Gehäuse 211 mit einem darin gebildeten Hohlraum 204, in dem die in Figur 1 dargestellte Energiequelle 100 aufgenommen ist. Mit Ausnahme seiner im Folgenden detailliert beschriebenen Bauteile ist der Schraubendreher 200 nur schematisch dargestellt. Weiterhin enthält der Schraubendreher 200 auch einen Deckel 203, der zum Halten der Energiequelle 100 innerhalb des Hohlraumes 204 und auch zum Verschliessen dieses Hohlraumes 204 dient. Der Schraubendreher 200 kann beispielsweise eine Länge von 17,5 cm und einen dazu senkrechten Durchmesser von 2,2 cm haben. Die genannten Abmessungen erlauben es, den Schraubendreher 200 einhändig zu halten und zu bedienen. Kleinere oder grössere, dann unter Umständen nur noch beidhändig bedienbare Schraubendreher, sind ebenfalls denkbar. Die Energiequelle 100 und der Schraubendreher 200 bilden zusammen ein erfindungsgemässes Werkzeugset.

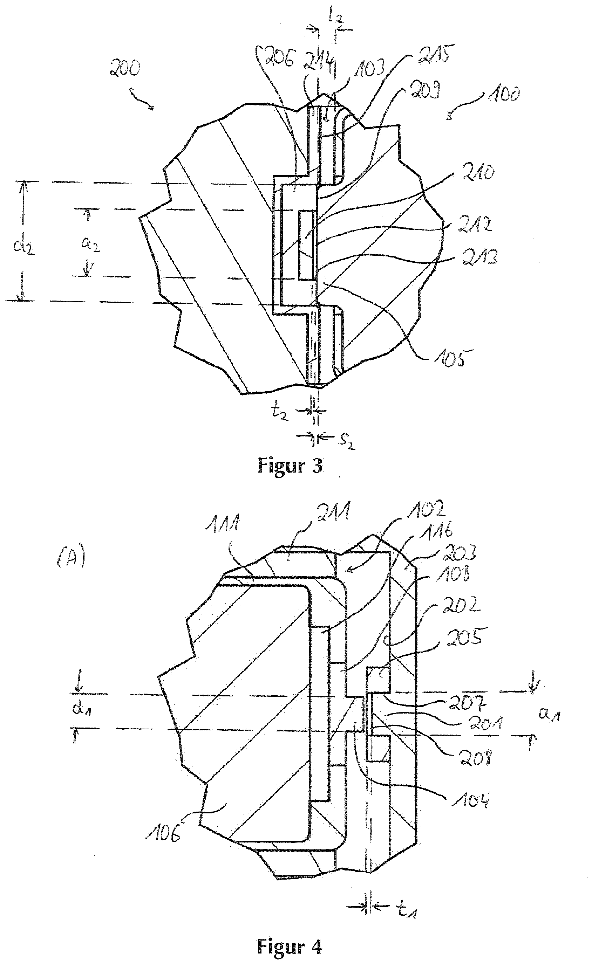

Figur 3 enthält eine Detailansicht des in Figur 2 mit X markierten Ausschnittes. Der Schraubendreher 200 enthält ein zweites Abgreifelement 206 mit einer ringförmigen, dem Hohlraum 204 zugewandten zweiten Abgreiffläche 209. Das zweite Abgreifelement 206 ist derart innerhalb eines Ringisolators 214 mit einer ringförmigen Isolatorfläche 215 angeordnet, dass die zweite Abgreiffläche 209 gegenüber der Isolatorfläche 215 um einen Abstand s₂ versenkt ist. Der Innendurchmesser der Abgreiffläche a₂ = 4 mm ist kleiner als der zweite Durchmesser d₂ des zweiten Poles 105, jedoch grösser als der erste Durchmesser d₁ des ersten Poles 104 der Energiequelle 100. Ferner ist der Abstand s₂ kleiner als die Länge l₂ des zweiten Poles 105. Dies erlaubt einen elektrischen Kontakt zwischen dem zweiten Pol 105 und dem zweiten Abgreifelement 206.

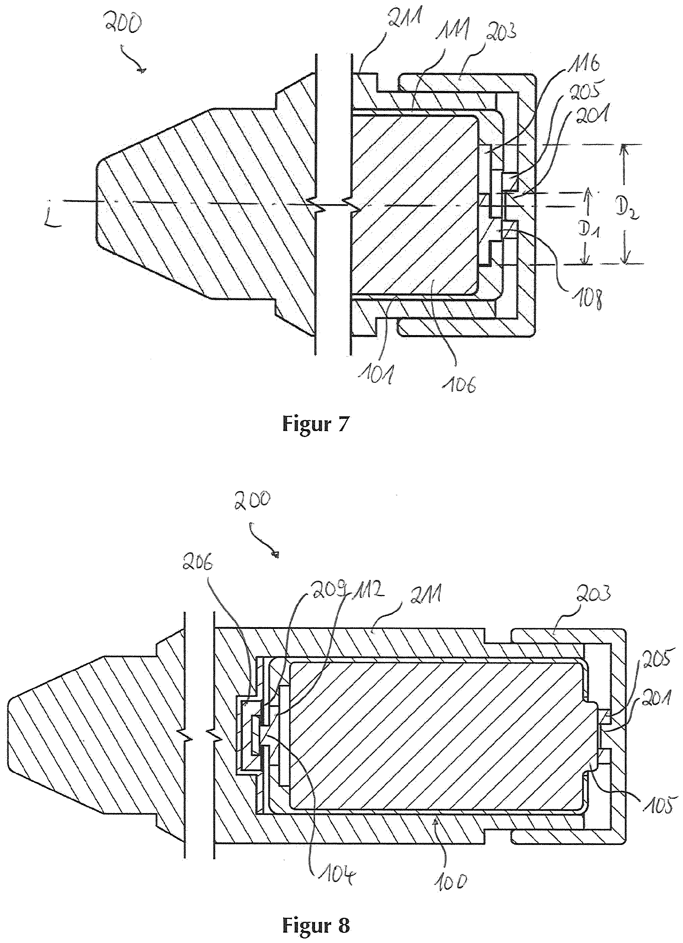

Das zweite Abgreifelement 206 umschliesst ringförmig einen zweiten Isolator 210 und überragt diesen in Längsrichtung des Schraubendrehers 200. Auf diese Weise wird ein zweiter Hohlraum 212 gebildet, der durch eine Innenwand 213 des zweiten Abgreifelementes 206 und den zweiten Isolator 210 begrenzt wird. Der zweite Hohlraum 212 verfügt entlang der Längsachse L der Energiequelle 100 über eine zweite Tiefe t₂ = 0,2 mm. Die weiteren Eigenschaften und Vorteile dieses zweiten Hohlraumes 212 werden unten im Zusammenhang mit Figur 8 noch erläutert.

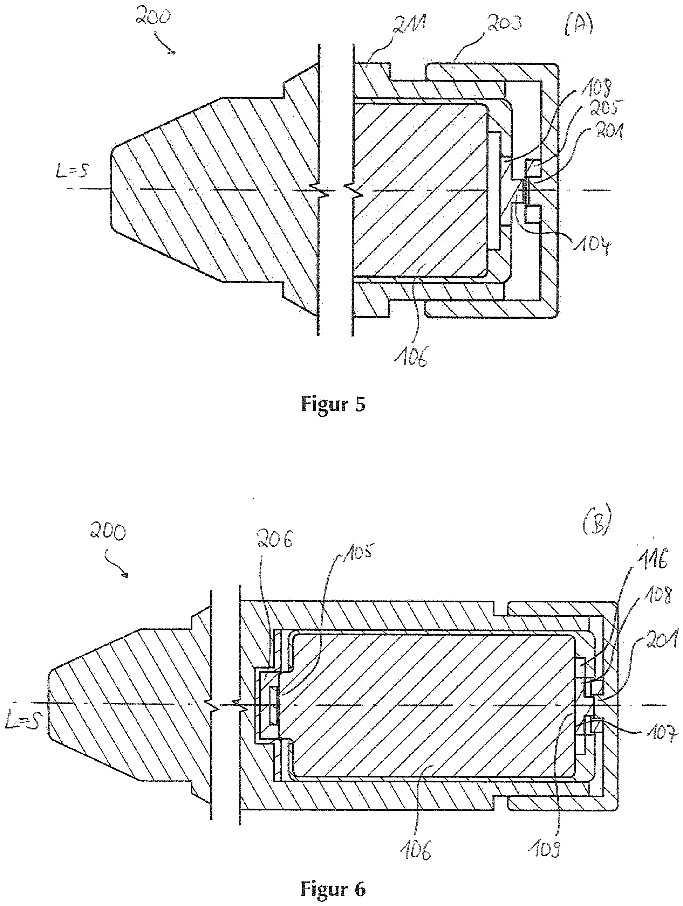

Die Detailansicht in Figur 4 zeigt den mit Y in Figur 2 dargestellten Ausschnitt. An einer Innenseite 202 des Deckels 203 sind ein erstes Abgreifelement 201 und ein erster elektrischer Isolator 205 angeordnet, welcher das erste Abgreifelement 201 ringförmig umschliesst und in Längsrichtung des Schraubendrehers 200 überragt. Auf diese Weise entsteht ein durch eine Innenwand 207 des ersten Isolators 205 und das erste Abgreifelement 201 teilweise begrenzter erster Hohlraum 208. Dieser erste Hohlraum 208 ist so bemessen, dass der erste Pol 104 der Energiequelle 100 im ersten Hohlraum 208 aufnehmbar und in Kontakt mit dem ersten Abgreifelement 201 bringbar ist. Hierzu ist der Innendurchmesser a₁ = 2,5 mm des ersten Isolators 205 grösser als der erste Durchmesser d₁ des ersten Poles 104, jedoch kleiner als der zweite Durchmesser d₂ des zweiten Poles 105 der Energiequelle. Zudem ist die Tiefe t₁ des ersten Hohlraums 208 kleiner als die Länge l₁ des ersten Poles 104. Dieser Kontakt entsteht jedoch erst in der in Figur 6 dargestellten Betriebsposition B (siehe dazu unten).

Figur 5 zeigt nochmals eine Detailansicht in der Bereitschaftsposition A. Durch Aufsetzen des Deckels 203 auf das Gehäuse 211 des Schraubendrehers 200 dringt der erste Pol 104 der Energiequelle 100 in den ersten Hohlraum 208 ein und gerät in Kontakt mit dem ersten Abgreifelement 201. Wird der Deckel 203 weiter in Richtung auf das Gehäuse 211 gedrückt, so übt das erste Abgreifelement 201 eine Kraft auf das Kontaktelement 108 aus, wodurch dieses von der in Figur 5 gezeigten Bereitschaftsposition A in die in Figur 6 dargestellte Betriebsposition B bewegt wird. Diese Bewegung erfolgt entlang einer Symmetrieachse S der Haltefläche 113, die mit der Längsachse L des Grundkörpers 101 der Energiequelle 100 übereinstimmt. In anderen Ausführungsbeispielen ist es allgemeiner auch denkbar, dass nur eine Komponente der Bewegung entlang der Symmetrieachse S der Haltefläche 113 verläuft. Die Tiefe t₁ des Hohlraums 208 ist so bemessen, dass beim Aufsetzen des Deckels 203 das Kontaktelement 108 zwar zwischenzeitlich lose, aber immer im ersten Isolator 205 geführt ist. Somit kann die mechanische Führung nur enden, wenn der Deckel 203 wieder vom Kontaktelement 108 wegbewegt wird.

In der Betriebsposition B steht der erste Kontakt 109 des Kontaktelements 108 in elektrischer Verbindung mit dem inneren Kontakt 107. Auf diese Weise ist auch das erste Abgreifelement 201 leitend mit dem inneren Kontakt 107 verbunden. Zudem ist auch der zweite Pol 105 der Energiequelle 100 in elektrischem Kontakt mit dem zweiten Abgreifelement 206. Somit liegt insgesamt die von der Spannungsquelle 106 erzeugte elektrische Spannung zwischen dem ersten Abgreifelement 201 und dem zweiten Abgreifelement 206 an, wodurch der Schraubendreher 200 antreibbar ist. In der Betriebsposition B ist das Kontaktelement 108 in Richtung der Längsachse L zwischen dem inneren Kontakt 107 und dem ersten Abgreifelement 201 eingeklemmt, kann also nicht innerhalb des Hohlraumes 116 verrutschen.

Wird der Deckel 203 wieder vom Gehäuse 211 gelöst, so wird hierdurch auch das erste Abgreifelement 201 vom Kontaktelement 108 entfernt. Hierdurch fällt die Klemmwirkung in Richtung der Längsachse L weg. Daher ist das Kontaktelement 108 in dieser in Figur 8 dargestellten Endposition C lose im Hohlraum 116 angeordnet. Hierdurch kann das Kontaktelement 108 innerhalb des Hohlraumes 116 in einer Ebene senkrecht zur Längsachse L verrutschen, insbesondere aufgrund der darauf einwirkenden Schwerkraft. Dies ist möglich, da der Hohlraum 116 senkrecht zur Längsachse L des Grundkörpers 101 einen Hohlraumdurchmesser D₂ aufweist, der grösser ist als der Haltekörperdurchmesser D₁. Die lose Anordnung wird weiterhin dadurch unterstützt, dass der Hohlraum 116 entlang der Längsachse L über eine Ausdehnung verfügt, die grösser ist als die entlang der Längsachse L des Grundkörpers 101 gemessene Höhe des Haltekörpers 112 des Kontaktelementes 108. Alternativ kann auch eine hier nicht dargestellte Feder vorgesehen sein, welche das Kontaktelement 108 in der Endposition C hält, in der es nicht mehr in elektrischen Kontakt mit dem ersten Abgreifelement 201 bringbar ist; hier ist das Kontaktelement 108 also nicht lose im Hohlraum 116 angeordnet.

Das Kontaktelement 108 kann also nicht mehr ohne weiteres in Kontakt mit dem ersten Abgreifelement 201 gebracht werden, so dass die Energiequelle 100 nicht nochmals derart in den Schraubendreher 200 eingesetzt werden kann, dass dieser von der Energiequelle 100 angetrieben werden kann. Überdies kann der Energiequelle 100 aufgrund der veränderten Position des Kontaktelementes 108 äusserlich angesehen werden, dass sie bereits in einem Werkzeug verwendet wurde.

In Figur 8 ist der gleiche Schraubendreher 200 mit der gleichen Energiequelle 100 dargestellt. Die Energiequelle 100 wurde hier jedoch in falscher Polung eingesetzt: Der erste Pol 104 befindet sich an der Seite des zweiten Abgreifelements 206, während sich der zweite Pol 105 an der Seite des ersten Abgreifelementes 201 befindet.

Allerdings besteht an beiden Seiten kein Kontakt zwischen Pol und Abgreifelement: Der erste Pol 104 hat einen Durchmesser d₁ (siehe Figur 1), der kleiner ist als der Innendurchmesser a₂ der zweiten Abgreiffläche 209 (siehe Figur 3). Zudem ist die erste Länge l₁ des erste Poles 104 (siehe Figur 1) grösser ist als die zweite Tiefe t₂ des zweiten Hohlraumes 212 (siehe Figur 3). Daher stösst der erste Pol 104 am zweiten Isolator 210 an, ohne dass dabei der Haltekörper 112 in Kontakt mit der zweiten Abgreiffläche 209 gelangen kann. Auf der anderen Seite ist der Durchmesser d₂ des zweiten Poles 105 (siehe Figur 1) grösser als der Innendurchmesser a₁ des ersten Isolators 205 (siehe Figur 4). Daher kann der zweite Pol 105 nicht in Kontakt mit dem versenkt angeordneten ersten Abgreifelement 201 gelangen.

Die Versenkung der zweiten Abgreiffläche 209 gegenüber der Isolatorfläche 215 (siehe Figur 3) verhindert, dass eine Energiequelle mit einem Pol, der sich jedoch nicht vom Grundkörper weg erstrecket, derart einsetzbar ist, dass dieser Pol in Kontakt mit der zweiten Abgreiffläche 209 gerät. Im Zusammenhang mit den weiteren obigen Ausführungen ergibt sich, dass eine Energiequelle mit zwei Polen, von denen sich aber höchstens einer vom Grundkörper weg erstreckt, überhaupt nicht in Kontakt mit beiden Abgreifelementen 201, 206 bringbar ist, und zwar in keiner der beiden Orientierungen.

Die Figuren 9 und 10 zeigen schematisch eine Energiequelle 100'. Diese enthält einen Grundkörper 101', der sich entlang einer Längsachse L' erstreckt und ein erstes Ende 102' sowie ein dem ersten Ende 102' bezüglich der Längsachse L' gegenüberliegendes zweites Ende 103' aufweist. Vom ersten Ende 102' erstreckt sich ein erster kreiszylinderförmiger Pol 104' mit einem Durchmesser d₁ weg, und vom zweiten Ende 103' erstreckt sich ein zweiter kreiszylinderförmiger Pol 105' mit einem zweiten Durchmesser d₂ weg, der grösser ist als der erste Durchmesser d₁. Im Gegensatz zum ersten in den Figuren 1 bis 8 dargestellten Ausführungsbeispiel enthält die Energiequelle 100' kein bewegliches Kontaktelement. Stattdessen ist der erste Pol 104' unbeweglich am Grundkörper 101' angeordnet.

Weiterhin sind in den Figuren 9 und 10 schematisch ein erstes kreiszylinderförmiges Abgreifelement 201, ein erster Isolator 205 sowie ein ringförmiges zweites Abgreifelement 206 dargestellt, welches einen zweiten Hohlraum 212 umschliesst. Der erste Isolator 205 umschliesst das erste Abgreifelement 201 ringförmig und überragt dieses in Richtung auf das zweite Abgreifelement 206. Der erste Isolator 205 hat einen Innendurchmesser a₁, der grösser ist als der Durchmesser d₁ des ersten Poles 104', jedoch kleiner als der Durchmesser d₂ des zweiten Poles 105'. Weiterhin ist die entlang der Längsachse L' gemessene erste Länge l₁ des ersten Poles 104' grösser als die in dieser Richtung gemessene erste Tiefe t₁ eines ersten Hohlraumes 208, der durch das erste Abgreifelement 201 und eine Innenwand 207 des ersten Isolators 205 begrenzt ist. Der Innendurchmesser a₂ des zweiten Abgreifelementes 206 ist grösser als der Innendurchmesser a₁ des ersten Isolators 105 und folglich ebenfalls grösser als der Durchmesser d₁ des ersten Poles 104', aber kleiner als der Durchmesser d₂ des zweiten Poles 105'. Insgesamt ist also d₁ < a₁ < a₂ < d₂.

In Figur 9 ist die Energiequelle 100' in korrekter Polung orientiert. Werden die Energiequelle 100' und der erste Isolator 205 zusammen mit dem ersten Abgreifelement 201 in Richtung auf das zweite Abgreifelement 206 bewegt, so dringt der erste Pol 104' aufgrund der oben beschriebenen Dimensionen in den ersten Hohlraum 208 ein, so dass er in Kontakt mit dem ersten Abgreifelement 201 gelangt, und der zweite Pol 105' liegt am zweiten Abgreifelement 206 an. Auf diese Weise liegt also zwischen den beiden Abgreifelementen 201 und 206 die von der Energiequelle 100' erzeugte Spannung an.

In Figur 10 ist die Energiequelle 100' in falscher Polung orientiert. Hier dringt der erste Pol 104' in den im zweiten Abgreifelement 206 gebildeten zweiten Hohlraum 212 ein. Da jedoch der Durchmesser d₁ des ersten Poles kleiner ist als der Innendurchmesser a₂, entsteht hier kein elektrischer Kontakt. Da zudem der zweite Durchmesser d₂ grösser ist als der Innendurchmesser a₁ des ersten Isolators 205, kann der zweite Pol 105' nicht im Kontakt mit dem ersten Abgreifelement 201 gelangen.

Eine (nicht erfindungsgemässe) Energiequelle mit zwei Polen identischer Durchmesser d₁ = d₂ würde keinen solchen Verpolungsschutz gewährleisten: Denn dann wären entweder die beiden identischen Durchmesser d₁ = d₂ der Pole beide kleiner als der grössere Innendurchmesser a₂, so dass keiner der beiden Pole in Kontakt mit dem zweiten Abgreifelement 206 gelangen könnte; oder aber die beiden identischen Durchmesser d₁ = d₂ der Pole wären mindestens so gross wie der grössere Innendurchmesser a₂, also grösser als der kleinere Innendurchmesser a₁, wobei dann allerdings keiner der beiden Pole in Kontakt mit dem ersten Abgreifelement 201 gelangen könnte, da sie beide am ersten Isolator 205 anstiessen. Eine solche Energiequelle wäre für den Anwender darüber hinaus schwierig richtig einzusetzen, da sich die beiden Pole optisch nicht unterscheiden.

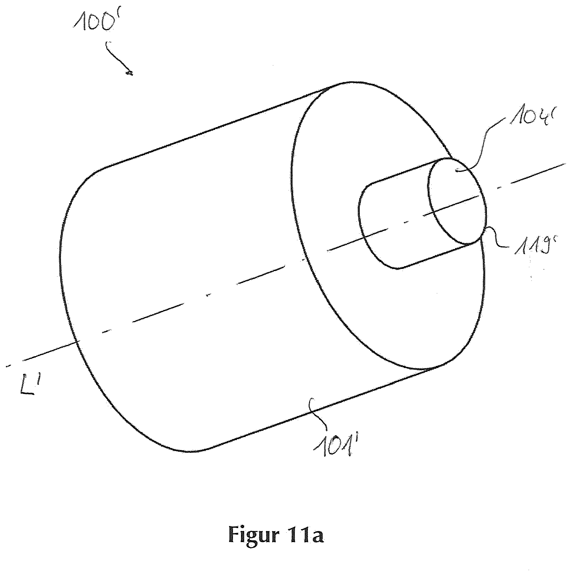

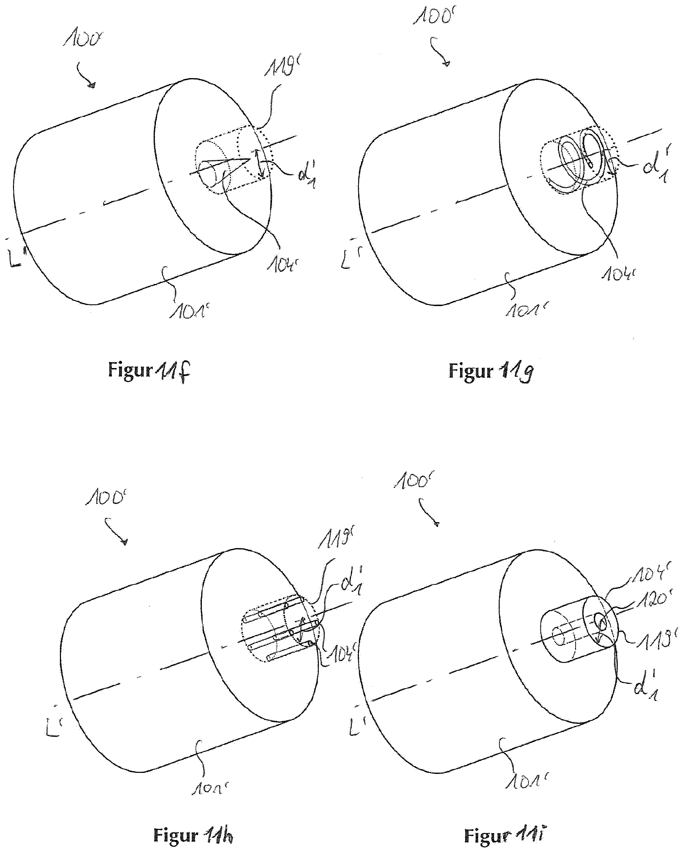

Die Figuren 11a bis 11i zeigen schematisch mehrere weitere Energiequellen 100', von denen jeweils nur der erste Pol 104' sichtbar ist. Die ersten Pole 104' aller in den Figuren 11a bis 11i dargestellten Energiequellen 100' haben den gleichen Durchmesser d₁'. Die Figuren 11a bis 11i zeigen jeweils einen imaginären Hohlzylinder 119', dessen Längsachse mit der Längsachse des Grundkörpers 101' übereinstimmt. Gezeichnet ist jeweils der kleinste solche imaginäre Hohlzylinder 119', in dem der erste Pol 104' vollständig aufnehmbar ist. Gemäss der hier verwendeten Definition ist der Durchmesser d₁' des ersten Poles 104' der Durchmesser dieses minimalen imaginären Hohlyzlinders 119'.

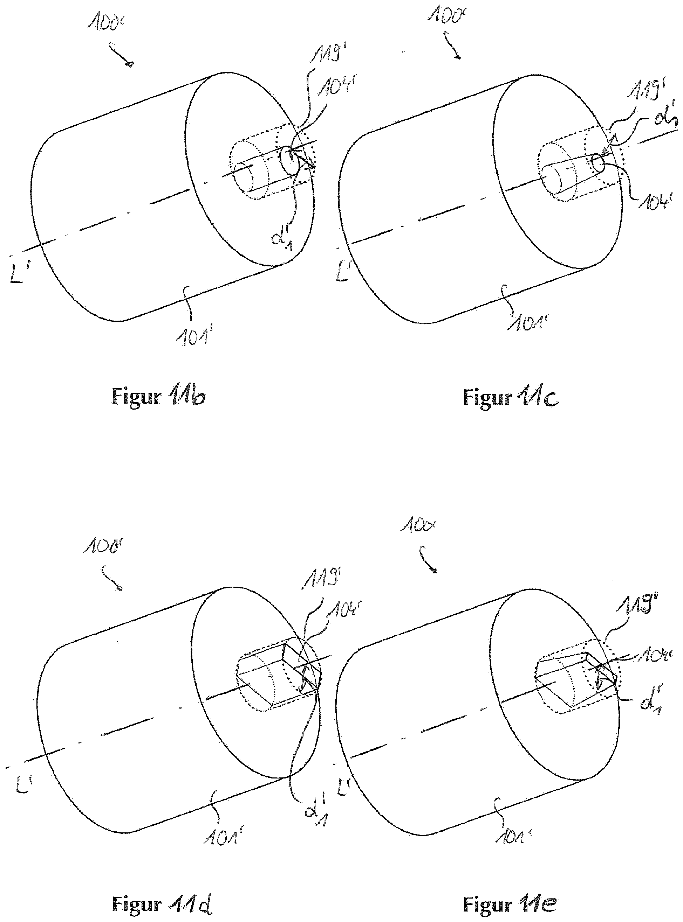

Im Einzelnen ist in Figur 11a der erste Pol 104' kreiszylinderförmig und entlang der Längsachse L' des Grundkörpers 101' angeordnet. Der erste Pol 104' stimmt hier also mit dem imaginären Hohlzylinder 119' überein und ist identisch mit dem in den Figuren 9 und 10 dargestellten. Der erste Pol 104' hat gemäss Figur 11b die Form eines Kreiszylinders, dessen Symmetrieachse jedoch gegenüber der Längsachse L' des Grundkörpers 101' exzentrisch verschoben ist. Der erste Pol 104' gemäss Figur 11c ist ebenfalls exzentrisch angeordnet, hat jedoch die Form eines Kreiskegelstumpfes. Der in Figur 11d abgebildete erste Pol 104' besitzt die Form eines Quaders. Ein erster Pol 104' in Form eines Kegelstumpfes mit rechteckiger, aber nicht quadratischer Grundfläche ist in Figur 11e dargestellt. Figur 11f zeigt einen ersten Pol 104' in Form eines exzentrischen Kreiskegels. Die in Figur 11g dargestellte Ausführungsform enthält einen ersten Pol 104', der die Form einer Helix aufweist und z.B. zusätzlich als Feder ausgeführt sein kann. Das Ausführungsbeispiel gemäss Figur 11g enthält insgesamt fünf erste Pole 104', die jeweils stabförmig ausgebildet sind und in Umfangsrichtung um die Längsachse L' angeordnet sind. Schliesslich zeigt Figur 11h einen ersten Pol 104', der die Form eines Hohlzylinders mit zentrischer Bohrung 120' aufweist. Selbstverständlich sind auch beliebige Kombinationen der oben genannten Ausführungsbeispiele denkbar.

## Patentansprüche

1. Elektrische Energiequelle (100) für ein elektrisch antreibbares Werkzeug (200), insbesondere ein elektrisch antreibbares chirurgisches Werkzeug (200), insbesondere einen elektrisch antreibbaren chirurgischen Schraubendreher (200), enthaltend
- ein Gehäuse (111), welches insbesondere zumindest teilweise einen Grundkörper (101) der Energiequelle (100) bildet,
- mindestens eine im Gehäuse (111) angeordnete Spannungsquelle (106) zum Erzeugen einer elektrischen Spannung,
- mindestens einen zweiten Pol (105) sowie
- mindestens einen inneren elektrischen Kontakt (107),
wobei die von der Spannungsquelle (106) erzeugte Spannung zwischen dem zweiten Pol (105) und dem inneren elektrischen Kontakt (107) anliegt oder anlegbar ist;
**gekennzeichnet durch**
mindestens ein elektrisches Kontaktelement (108) mit einem ersten elektrischen Kontakt (109) und einem zweiten elektrischen Kontakt (110), welche elektrisch miteinander verbunden sind, wobei
- das Kontaktelement (108) einen ersten Pol (104) enthält, der den zweiten Kontakt (110) enthält;
- sich das Kontaktelement (108) in einer Bereitschaftsposition (A) befindet, in der es lösbar direkt oder indirekt mit dem Gehäuse (111) verbunden ist und in der weder der erste Kontakt (109) noch der zweite Kontakt (110) in elektrischer Verbindung mit dem inneren Kontakt (107) steht;
- das Kontaktelement (108) durch Einwirkung einer Kraft, welche durch ein erstes Abgreifelement (201) des Werkzeuges (200) ausübbar ist, von der Bereitschaftsposition (A) in eine Betriebsposition (B) bewegbar ist, in der der erste Kontakt (109) in elektrischer Verbindung mit dem inneren Kontakt (107) steht, so dass die von der Spannungsquelle (106) erzeugte Spannung zwischen dem zweiten Pol (105) und dem ersten Abgreifelement (201) anliegt;
- sich das Kontaktelement (108) nach erstmaligem Entfernen des ersten Abgreifelementes (201) aus der Betriebsposition (B) in eine Endposition (C) bewegt, in der das Kontaktelement (108) nicht mehr in elektrischen Kontakt mit dem ersten Abgreifelement (201) bringbar ist, so dass die von der Spannungsquelle (106) erzeugte Spannung nicht mehr zwischen dem zweiten Pol (105) und dem ersten Abgreifelement (201) anlegbar ist.

2. Energiequelle (100) gemäss Anspruch 1,
**dadurch gekennzeichnet, dass**
das Kontaktelement (108) in der Bereitschaftsposition (A) am Gehäuse (111) eingeklemmt, eingeklebt, eingepresst oder eingeschweisst ist oder über mindestens eine Sollbruchstelle mit dem Gehäuse (111) verbunden ist.

3. Energiequelle (100) gemäss einem der Ansprüche 1 und 2,
**dadurch gekennzeichnet, dass**
das Kontaktelement (108) eine Haltefläche (113) aufweist und das Gehäuse (111) eine Gegenhaltefläche (114) aufweist, wobei das Kontaktelement (108) in der Bereitschaftsposition (A) durch mechanischen Kontakt der Haltefläche (113) mit der Gegenhaltefläche (114) haltbar ist.

4. Energiequelle (100) gemäss Anspruch 3,
**dadurch gekennzeichnet, dass**
das Kontaktelement (108) entlang einer Längsachse, insbesondere einer Symmetrieachse (S) der Haltefläche (113) von der Bereitschaftsposition (A) in die Betriebsposition (B) bewegbar ist, wobei bevorzugt die Längsachse der Haltefläche (113) mit der Längsachse (L) des Grundkörpers (101; 101') übereinstimmt.

5. Energiequelle (100) gemäss einem der Ansprüche 1 bis 4,
**dadurch gekennzeichnet, dass**
sie mindestens einen Hohlraum (116) enthält, in dem das Kontaktelement (108) in der Endposition (C) lose angeordnet ist.

6. Energiequelle (100) gemäss Anspruch 5,
**dadurch gekennzeichnet, dass**
der Hohlraum (116) im Wesentlichen kreiszylinderförmig ausgebildet ist und senkrecht zur Längsachse (L) des Grundkörpers (101) einen Hohlraumdurchmesser (D₂) aufweist, der grösser ist als ein Haltekörperdurchmesser (D₁) eines Haltekörpers (112) des Kontaktelements (108).

7. Energiequelle (100) gemäss einem der Ansprüche 1 bis 6,
**dadurch gekennzeichnet, dass**
das erste Abgreifelement (201) an einer Innenseite (202) eines Deckels (203) des Werkzeuges (200) angeordnet ist, wobei der Deckel (203) zum Halten der Energiequelle (100) innerhalb eines Hohlraumes (204) des Werkzeuges (200) und optional zum Verschliessen dieses Hohlraumes (204) ausgebildet ist.

8. Werkzeugset, enthaltend
- mindestens ein elektrisch antreibbares Werkzeug (200), insbesondere mindestens ein elektrisch antreibbares chirurgisches Werkzeug (200), insbesondere mindestens einen elektrisch antreibbaren chirurgischen Schraubendreher (200),
- mindestens eine elektrische Energiequelle (100; 100') gemäss einem der vorangehenden Ansprüche, mittels deren das Werkzeug (200) elektrisch antreibbar ist.

9. Werkzeugset gemäss Anspruch 8,
**dadurch gekennzeichnet, dass**
das Werkzeug (200) ein erstes Abgreifelement (201) aufweist, welches derart ausgebildet und angeordnet ist, dass das Kontaktelement (108) der Energiequelle (100) durch Einwirkung einer Kraft, welche durch das erste Abgreifelement (201) ausübbar ist, von der Bereitschaftsposition (A) in die Betriebsposition (B) bewegbar ist.

10. Werkzeugset gemäss einem der Ansprüche 8 und 9,
**dadurch gekennzeichnet, dass**
die Energiequelle (100; 100') gemäss einem der Ansprüche 1 und 2 ausgebildet ist und das Werkzeug (200) ein erstes Abgreifelement (201) und ein zweites Abgreifelement (206) zum Abgreifen der zwischen dem ersten Pol (104; 104') und dem zweiten Pol (105; 105') anliegenden oder anlegbaren elektrischen Spannung aufweist, wobei die Energiequelle (100; 100') und das Werkzeug (200) derart ausgebildet und aufeinander abgestimmt sind, dass
- gleichzeitig der erste elektrische Pol (104; 104') mit dem ersten Abgreifelement (201) in Kontakt bringbar ist und der zweite elektrische Pol (105; 105') mit dem zweiten Abgreifelement (206) in Kontakt bringbar ist und
- der erste elektrische Pol (104; 104') nicht mit dem zweiten Abgreifelement (206) in Kontakt bringbar ist und/oder
der zweite elektrische Pol (105; 105') nicht mit dem ersten Abgreifelement (201) in Kontakt bringbar ist.

11. Werkzeugset gemäss einem der Ansprüche 8 bis 10,
**dadurch gekennzeichnet, dass**
das Werkzeug (200) einen Hohlraum (204) zum Aufnehmen der Energiequelle (100; 100') sowie einen Deckel (203) enthält, an dessen Innenseite (202) das erste Abgreifelement (201) angeordnet ist und der zum Halten der Energiequelle (100; 100') innerhalb des Hohlraumes (204) und zum Verschliessen dieses Hohlraumes (204) ausgebildet ist.

12. Werkzeugset gemäss einem der Ansprüche 10 und 11,
**dadurch gekennzeichnet, dass**
das Werkzeug (200), insbesondere der Deckel (203) des Werkzeuges (200), einen ersten elektrischen Isolator (205) enthält, welcher das erste Abgreifelement (201) ringförmig umschliesst und in Längsrichtung des Werkzeuges (200) überragt, so dass ein durch eine Innenwand (207) des ersten Isolators (205) und das erste Abgreifelement (201) teilweise begrenzter erster Hohlraum (208) entsteht, der so bemessen ist, dass nur der erste Pol (104) der Energiequelle (100) in dem ersten Hohlraum (208) aufnehmbar und in Kontakt mit dem ersten Abgreifelement (201) bringbar ist, nicht aber auch der zweite Pol (105).

13. Verfahren zum Einsetzen einer Energiequelle (100) gemäss einem der Ansprüche 1 bis 7 in ein elektrisch antreibbares Werkzeug (200), insbesondere ein elektrisch antreibbares chirurgisches Werkzeug (200), insbesondere einen elektrisch antreibbaren chirurgischen Schraubendreher (200), enthaltend einen Schritt, in dem das Kontaktelement (108) der Energiequelle (100) durch Einwirkung einer Kraft durch das erste Abgreifelement (201) des Werkzeuges (200) von der Bereitschaftsposition (A) in die Betriebsposition (B) bewegt wird.

## Claims

1. An electrical energy source (100) for an electrically drivable tool (200), in particular an electrically drivable surgical tool (200), in particular an electrically drivable surgical screwdriver (200), comprising
- a housing (111) that in particular, at least partly, forms a main body (101) of the energy source (100),
- at least one voltage source (106), arranged in the housing (111), for generating an electrical voltage,
- at least one second terminal (105), and
- at least one inner electrical contact (107),
wherein the voltage generated by the voltage source (106) is applied or can be applied between the second terminal and (105) the inner electrical contact (107);
which comprises
at least one electrical contact element (108) having a first electrical contact (109) and a second electrical contact (110) that are electrically connected to each other, wherein
- the contact element (108) comprises a first terminal (104), which comprises the second contact (110);
- the contact element (108) is in a standby position (A), in which it is releasably connected, directly or indirectly, to the housing (111), and in which neither the first contact (109) nor the second contact (110) is electrically connected to the inner contact (107);
- by action of a force that can be exerted by a first tap-off element (201) of the tool (200), the contact element (108) can be moved from the standby position (A) into an operating position (B) in which the first contact (109) is electrically connected to the inner contact (107), such that the voltage generated by the voltage source (106) is applied between the second terminal (105) and the first tap-off element (201);
- after the first tap-off element (201) has been removed for the first time, the contact element (108) moves from the operating position (B) into an end position (C) in which the contact element (108) can no longer be brought into electrical contact with the first tap-off element (201), such that the voltage generated by the voltage source (106) can no longer be applied between the second terminal (105) and the first tap-off element (201) .

2. The energy source (100) as claimed in claim 1,
**characterized in that**
in the standby position (A) the contact element (108) may be clamped in, adhesive-bonded in, pressed in or welded in on the housing (111), or connected to the housing (111) via at least one predetermined breaking point.

3. The energy source (100) as claimed in either one of claims 1 and 2,
**characterized in that**
the contact element (108) has a holding surface (113), and the housing (111) has a counter-holding surface (114), wherein the contact element (108) can be held in the standby position (A) by mechanical contact of the holding surface (113) with the counter-holding surface (114).

4. The energy source (100) as claimed in claim 3,
**characterized in that**
the contact element (108) is movable along a longitudinal axis, in particular an axis of symmetry (S) of the holding surface (113), from the standby position (A) into the operating position (B), wherein, preferably, the longitudinal axis of the holding surface (113) coincides with the longitudinal axis (L) of the main body (101; 101'.

5. The energy source (100) as claimed in any one of claims 1 to 4,
**characterized in that**
it comprises at least one cavity (116), in which the contact element (108) is loosely arranged in the end position (C) .

6. The energy source (100) as claimed in claim 5,
**characterized in that**
the cavity (116) is realized substantially in the form of a circular cylinder and has a cavity diameter (D₂), perpendicular to the longitudinal axis (L) of the main body (101), that is greater than a diameter (D₁) of a holding body (112) of the contact element (108).

7. The energy source (100) as claimed in any one of claims 1 to 6,
**characterized in that**
the first tap-off element (201) is arranged on an inner side (202) of a cover (203) of the tool (200), wherein the cover (203) is designed to hold the energy source (100) inside the cavity (204) of the tool (200), and optionally to close this cavity (204).

8. A tool set, comprising
- at least one electrically drivable tool (200), in particular at least one electrically drivable surgical tool (200), in particular at least one electrically drivable surgical screwdriver (200),
- at least one electrical energy source (100; 100') as claimed in any one of the preceding claims, by means of which the tool (200) is electrically drivable.

9. The tool set as claimed in claim 8,
**characterized in that**
the tool (200) has a first tap-off element (201), which is realized and arranged in such a manner that the contact element (108) of the energy source (100) can be moved from the standby position (A) into the operating position (B) by action of a force that can be exerted by the first tap-off element (201).

10. The tool set as claimed in either one of claims 8 and 9,
**characterized in that**
the energy source (100; 100') is realized as claimed in either one of claims 1 and 2, and the tool (200) has a first tap-off element (201) and a second tap-off element (206) for tapping off the voltage that is applied or can be applied between the first terminal (104; 104') and the second terminal (105; 105'), wherein the energy source (100; 100') and the tool (200) are realized and matched to each other in such a manner that
- simultaneously, the first electrical terminal (104; 104') can be brought into contact with the first tap-off element (201) and the second electrical terminal (105; 105') can be brought into contact with the second tap-off element (206), and
- the first electrical terminal (104; 104') cannot be brought into contact with the second tap-off element (206)
and/or
the second electrical terminal (105; 105') cannot be brought into contact with the first tap-off element (201) .

11. The tool set as claimed in any one of claims 8 to 10,
**characterized in that**
the tool (200) comprises a cavity (204) for accommodating the energy source (100; 100'), and a cover (203), on the inner side (202) of which the first tap-off element (201) is arranged and which is designed to hold the energy source (100; 100') inside the cavity (204) and to close this cavity (204).

12. The tool set as claimed in either one of claims 10 and 11,
**characterized in that**
the tool (200), in particular the cover (203) of the tool (200), comprises a first electrical insulator (205) that encompasses the first tap-off element (201) in the manner of a ring and projects over it in the longitudinal direction of the tool (200), such that there is produced a first cavity (208), which is partly delimited by an inner wall (207) of the first insulator (205) and the first tap-off element (201), and which is dimensioned such that only the first terminal (104) of the energy source (100) can be accommodated in the first cavity (208) and brought into contact with the first tap-off element (201), but not also the second terminal (105).

13. A method for inserting an energy source (100) as claimed in any one of claims 1 to 7 into an electrically drivable tool (200), in particular an electrically drivable surgical tool (200), in particular an electrically drivable surgical screwdriver (200), comprising a step in which, by action of a force by the first tap-off element (201) of the tool (200), the contact element (108) of the energy source (100) is moved from the standby position (A) into the operating position (B).

## Revendications

1. Source d'énergie électrique (100) pour un outil (200) entrainable électriquement, en particulier un outil chirurgical (200) entrainable électriquement, en particulier un tournevis chirurgical (200) entrainable électriquement, comprenant
- un boîtier (111), qui forme notamment au moins partiellement un corps de base (101) de la source d'énergie (100),
- au moins une source de tension (106) arrangée dans le boîtier (111) pour générer une tension électrique,
- au moins un deuxième pôle (105) et
- au moins un contact électrique interne (107),
dans lequel la tension générée par la source de tension (106) entre le deuxième pôle (105) et le contact électrique interne (107) est appliquée ou peut être appliquée;
**caractérisé par**
au moins un élément de contact électrique (108) ayant un premier contact électrique (109) et un deuxième contact électrique (110), qui sont connectés électriquement l'un à l'autre, dans lequel
- l'élément de contact (108) comprend un premier pôle (104) qui contient le deuxième contact (110);
- l'élément de contact (108) est dans une position d'attente (A), dans laquelle il est connecté directement ou indirectement de manière amovible au boîtier (111) et dans lequel ni le premier contact (109) ni le deuxième contact (110) sont en connexion électrique avec le contact interne (107);
- l'élément de contact (108) peut être déplacé de la position d'attente (A) à une position de fonctionnement (B) par l'action d'une force, qui peut être exercée par un premier élément de prélèvement (201) de l'outil (200), dans lequel le premier contact (109) est en connexion électrique avec le contact interne (107), de telle sorte que la tension générée par la source de tension (106) est appliquée entre le deuxième pôle (105) et le premier élément de prélèvement (201);
- après le premier retrait du premier élément de prélèvement (201), l'élément de contact (108) se déplace de la position de fonctionnement (B) dans une position finale (C), dans laquel l'élément de contact (108) ne peut plus être mis en contact électrique avec le premier élément de prélèvement (201), de telle sorte que la tension générée par la source de tension (6) ne peut plus être appliquée entre le deuxième pôle (105) et le premier élément de prélèvement (201).

2. Source d'énergie (100) selon la revendication 1,
**caractérisée en ce**
**que**, en position d'attente (A)l'élément de contact (108) est serré, collé, pressé ou soudé au boîtier (111 ou connecté au boîtier (111) par au moins un point de rupture prédéterminé.

3. Source d'énergie (100) selon l'une quelconque des revendications 1 et 2,
**caractérisée en ce**
**que** l'élément de contact (108) présente une surface de support (113) et le boîtier (111) comporte une surface de contre-support (114), l'élément de contact (108) étant tenu, en position d'attente (A), par le contact mécanique entre la surface de support (113) et la surface de contre-support (114).

4. Source d'énergie (100) selon la revendication 3,
**caractérisée en ce**
**que** l'élément de contact (108) peut être déplacé, le long d'un axe longitudinal, en particulier un axe de symétrie (S) de la surface de support (113), de la position d'attente (A) dans la position de fonctionnement (B), dans lequel, de préférence, l'axe longitudinal de la surface de support (113) et l'axe longitudinal (L) du corps de base (101, 101') coïncident.

5. Source d'énergie (100) selon l'une quelconque des revendications 1 à 4,
**caractérisée en ce**
**qu'**elle comprend au moins une cavité (116) dans laquelle l'élément de contact (108), en position finale (C), est arrangé de manière lâche.

6. Source d'énergie (100) selon la revendication 5,
**caractérisée en ce**
**que** la cavité (116) est en forme de cylindre circulaire et a, perpendiculairement à l'axe longitudinal (L) du corps de base (101), un diamètre de cavité (D₂) supérieur à un diamètre (D₁) d'un corps de maintien de l'élément de contact (108).

7. Source d'énergie (100) selon l'une quelconque des revendications 1 à 6,
**caractérisée en ce**
**que** le premier élément de prélèvement (201) est arrangé sur un côté intérieur (202) d'un couvercle (203) de l'outil (200), le couvercle (203) étant formé pour tenir la source d'énergie (100) dans une cavité (204) de l'outil (200) et, éventuellement, pour fermer cette cavité (204).

8. Ensemble d'outils, comprenant
- au moins un outil (200) pouvant être entrainé électriquement, en particulier au moins un outil chirurgical (200) pouvant être entrainé électriquement, notamment au moins un tournevis chirurgical (200),
- au moins une source d'énergie électrique (100; 100') selon l'une quelconque des revendications précédentes au moyen de laquelle l'outil (200) peut être entrainée électriquement.

9. Ensemble d'outils selon la revendication 8,
**caractérisé en ce**
**que** l'outil (200) comporte un premier élément de prélèvement (201) conçu et arrangé de telle manière à ce que l'élément de contact (108) de la source d'énergie (100) soit soumis à l'action d'une force qui, par le premier élément de prélèvement (201), peut être déplacer de la position d'attente (A) à la position de fonctionnement (B).

10. Ensemble d'outils selon l'une quelconque des revendications 8 et 9,
**caractérisé en ce**
**que** la source d'énergie (100, 100') est formée selon l'une quelconque des revendications 1 et 2 et l'outil (200) comprend un premier élément de prélèvement (201) et un deuxième élément de prélèvement (206) pour prélever une tension électrique appliquée ou pouvant appliquée entre le premier pôle (104, 104') et le deuxième pôle (105, 105'), la source d'énergie (100, 100') et l'outil (200) étant conçus et adaptés l'un à l'autre de telle manière que
- simultanément, le premier pôle électrique (104, 104') peut être mis en contact avec le premier élément de prélèvement (201) et le deuxième pôle électrique (105, 105') peut être mis en contact avec le deuxième élément de prélèvement (206) et
- le premier pôle électrique (104, 104') ne peut pas être mis en contact avec le deuxième élément de prélèvement (206) et / ou le deuxième pôle électrique (105, 105') ne peut pas être mis en contact avec le premier élément de prélèvement (201).

11. Ensemble d'outils selon l'une quelconque des revendications 8 à 10,
**caractérisé en ce**
**que** l'outil (200) comprend une cavité (204) pour recevoir la source d'énergie (100, 100') et un couvercle (203) à l'intérieur (202) duquel le premier élément de prélèvement (201) est arrangé et qui est adapté pour maintenir la source d'énergie (100, 100') à l'intérieur de la cavité (204) et pour fermer cette cavité (204).

12. Ensemble d'outils selon l'une quelconque des revendications 10 et 11,
**caractérisé en ce**
**que** l'outil (200), en particulier le couvercle (203) de l'outil (200), contient un premier isolant électrique (205) qui entoure de manière annulaire le premier élément de prélèvement (201) et qui dépasse l'outil (200) dans la direction longitudinale de, de telle sorte qu'une première cavité (208) partiellement délimitée par une paroi interne (207) du premier isolant (205) et le premier élément de prélèvement (201), est dimensionnés de telle sorte que seulement le premier pôle (104) de la source d'énergie (100) peut être reçu dans la première cavité (208) et peut être mis en contact avec le premier élément de prélèvement (201), mais pas le deuxième pôle (105).

13. Procédé d'insertion d'une source d'énergie (100) selon l'une quelconque des revendications 1 à 7 dans un outil (200) entrainable électriquement, en particulier un outil chirurgical (200) pouvant entrainable électriquement, en particulier un tournevis chirurgical (200) entrainable électriquement, comprenant une étape dans laquelle l'élément de contact (108) de la source d'énergie (100) est déplacé de la position d'attente (A) à la position de fonctionnement (B) par l'action d'une force exercée par le premier élément de prélèvement (201) de l'outil (200).
